# EUROPEAN PATENT SPECIFICATION

(11) **EP 2 812 007 B1**
(45) Date of publication and mention of the grant of the patent: **30.12.2020**
(21) Application number: 13747028.2
(22) Date of filing: 08.02.2013
(51) Int. Cl.: A61K 31/70, A61K 31/505, A61K 9/16, A61K 9/14, A61P 1/12, A61K 31/198, A61K 45/06, A61K 33/00, A61K 33/06, A61K 33/20, A61K 31/205, A61K 31/405

(54) **COMPOSITIONS AND METHODS FOR TREATING DIARRHEA**
ZUSAMMENSTELLUNGEN UND VERFAHREN ZUR BEHANDLUNG VON DIARRHÖE
COMPOSITIONS ET MÉTHODES UTILISABLES EN VUE DU TRAITEMENT DE LA DIARRHÉE

(30) Priority: 08.02.2012 US 201261596480 P
(43) Date of publication of application: 17.12.2014
(73) Proprietor: University of Florida Research Foundation, Inc., Gainesville, FL 32611 (US)
(72) Inventor: VIDYASAGAR, Sadasivan, Gainesville, FL 32607 (US); OKUNIEFF, Paul, Gainesville, FL 32608 (US); ZHANG, Lurong, Gainesville, FL 32608 (US)
(74) Representative: Teuten, Andrew John
(86) International application number: PCT/US2013/025294
(87) International publication number: WO 2013/119917

(56) References cited:
- KR-A- 20060 130 583
- KR-A- 20070 103 506
- US-A1- 2003 143 293
- US-A1- 2012 077 748
- GUTIERREZ, C. ET AL.: 'Does an L-glutamine-containing, glucose-free, oral rehydration solution reduce stool output and time to rehydrate in children with acute diarrhoea? A double-blind randomized clinical trial' JOURNAL OF HEALTH, POPULATION AND NUTRITION vol. 25, no. 3, 2007, pages 278 - 284, XP055078283
- MATHEW, J. L.: 'Non-glucose oral rehydration solution - Does it make a good thing better?' INDIAN PEDIATRICS vol. 46, 2009, pages 501 - 505, XP055078300
- R. HAIDER ET AL: "Management of acute diarrhoea in diabetic patients using oral rehydration solutions containing glucose, rice, or glycine", BMJ, vol. 308, 5 March 1994 (1994-03-05), pages 624-626, XP008177144,

## Description

### BACKGROUND OF INVENTION

Rotavirus infection is the leading cause of severe diarrheal diseases and dehydration in infants and young children throughout the world. Symptoms of rotavirus infection include watery diarrhea, severe dehydration, fever, and vomiting. Rotavirus infection can also result in jejunal lesions with maximal damage occurring on day three post-inoculation, and in some instances, causing a reduction of villus surface area to 30% to 50% of normal (Rhoads et al. (1996) J. Diarrhoeal Dis. Res. 14(3):175-181).

The pathophysiological mechanism through which rotavirus induces diarrhea is via the action of an enterotoxin - non-specific protein-4 (NSP4) on small intestine epithelial cells. NSP4 mobilizes intracellular Ca²⁺ in both small and large intestinal crypt epithelia to mimic the secretory effects of the cholinergic agonist carbachol (CCh) in potentiating cAMP-dependent fluid secretion.

Increase in intracellular cAMP ([cAMP]ᵢ) and Ca²⁺ ([Ca²⁺]ᵢ are known to mediate Cl⁻ and/or HCO₃⁻ secretion in diarrhea associated with both infective as well as inflammatory conditions (Zhang et al. (2007) J Physiol 581(3):1221-1233). The osmotic gradient generated by the chloride secretion results in passive movement of water into the intestinal lumen, thereby causing a watery stool. Cl⁻ secretion with passive water movement occurs in lesser quantity during normal digestion and absorption, which is essential for proper mixing, churning and smooth propulsion through the gut lumen. In a normal absorptive small intestine, there is a fine balance between absorption occurring in the villus cell region and the secretion from the crypt cells. An imbalance resulting from a decreased absorption, increased secretion, or a combined effect can result in diarrhea.

Calcium activated chloride channels (CaCCs) are involved in important physiological processes. Transfection of epithelial cells with specific small interfering RNA against each of the membrane proteins that are regulated by IL-4 reveals that TMEM16A, a member of a family of putative plasma membrane protein with unknown function, is associated with calcium-dependent chloride current (Caputo et al. (2008) Science 322(5901):590-594). TMEM16A is widely expressed in mammalian tissues, including tracheal, intestinal, and glandular epithelia, smooth muscle cells, and interstitial cells of Cajal in the gastrointestinal tract (Namkung et al., J. Biol. Chem. 286(3):2365-2374).

Luminal glucose absorption by the enterocytes in the small intestine follows secondary active transport (Hediger et al. (1994) Physiol. Rev. 74(4):993-1026; Wright et al. (2004) Physiology (Bethesda) 19:370-376). The sodium-glucose transporter (SGLT-1) has a stoichiometry of 2:1, thereby transporting two sodium ions for one glucose molecule across the luminal membrane (Chen et al. (1995) Biophys. J. 69(6):2405-2414). The tightly coupled sodium glucose transport is driven by the electrochemical gradient of Na⁺ formed by Na-K-ATPase activity. The SGLT-1-mediated, electrogenic Na⁺ absorption causes solvent drag, thereby leading to passive absorption of water from the lumen.

Maintenance of hydration is a critical element in the treatment of diarrheal diseases including rotavirus-induced diarrhea. Currently, secretory diarrhea is treated with an oral rehydration drink (ORD) - a salt solution containing sodium and a significant amount of glucose and other sugar molecules. Glucose has always been a mainstay in both enteral and parenteral fluids for correcting electrolyte and nutrient absorption defects associated with disease conditions. ORDs are designed to correct the loss of fluids and electrolytes in secretory diarrhea, based on the theory that upon the active, coupled uptake of sodium and glucose in the small intestine, there is a subsequent influx of water that follows the movement of absorbed state.

Although ORDs provide a significant breakthrough in the treatment of cholera and other diarrheal conditions, there is a need to improve its efficiency. Improved formulation is needed due to the poor rate of rehydration provided by existing ORD formulations. The rate of rehydration in diarrheal patients is not in step with the rate of electrolyte loss. The existing ORD formulations have been shown to be ineffective in treating rotavirus-induced diarrhea, while the exact cause for the ineffectiveness remains unknown. Accordingly, a need exists for improved ORD formulations for treatment of diarrhea.

US2012/077748A1 (Vidyasagar Sadasivan et al.) discloses therapeutic compositions said to be of use in the treatment or amelioration of injury to small intestine mucosa. In preferred embodiments, the composition comprises one or more nutrients and/or electrolytes that acquire or retain considerable absorptive capacity.

Gutiérrez C. et al. (Journal of Health, Population and Nutrition, vol. 25, no. 3, 2007, pages 278-284) describe a study investigating whether an oral rehydration solution (ORS) in which glucose is replaced by L-glutamine (L-glutamine ORS) is more effective than the standard glucose-based rehydration solution recommended by the World Health Organization (WHO-ORS) in reducing the stool volume and time to rehydrate in acute diarrhea.

Haider R. et al. (BMJ, vol. 308, 1994, pages 624-626) describe a study assessing the risk of hyperglycaemia with two standard oral rehydration solutions that contain carbohydrate compared with a carbohydrate free solution during rehydration of diabetic patients with acute diarrhoea. The study concludes that oral rehydration solutions containing glucose, rice powder, or glycine can be safely administered to diabetic patients with acute diarrhoea and some dehydration.

### BRIEF SUMMARY

The present invention provides therapeutic compositions for use in methods for treating diarrhea, for providing rehydration, for correcting electrolyte and fluid imbalances, and/or for improving small intestine function.

In one embodiment of the disclosure is provided a composition formulated for enteral administration, wherein the composition does not contain glucose. In a preferred embodiment, the composition is formulated as an oral rehydration drink (ORD). In another preferred embodiment, the composition is in a powder form, and can be reconstituted in water for use as an ORD.

In one embodiment, the composition of the present disclosure comprises one or more ingredients selected from free amino acids; electrolytes; di-peptides and/or oligo-peptides; vitamins; and optionally, water, therapeutically acceptable carriers, excipients, buffering agents, flavoring agents, colorants, and/or preservatives. In one embodiment, the total osmolarity of the composition is from about 100 mosm to 250 mosm. In one embodiment, the composition has a pH from about 2.9 to 7.3.

In a further embodiment, the present disclosure provides a treatment comprising administering, via an enteral route, to a subject in need of such treatment, an effective amount of a composition of the disclosure. The composition can be administered once or multiple times each day. In a preferred embodiment, the composition is administered orally.

In a preferred embodiment, the present invention provides treatment of diarrhea induced by rotavirus infection and/or NSP4. In another preferred embodiment, the present disclosure results in decreased Cl⁻ and/or HCO₃⁻ secretion and/or improved fluid absorption.

### BRIEF DESCRIPTION OF DRAWINGS

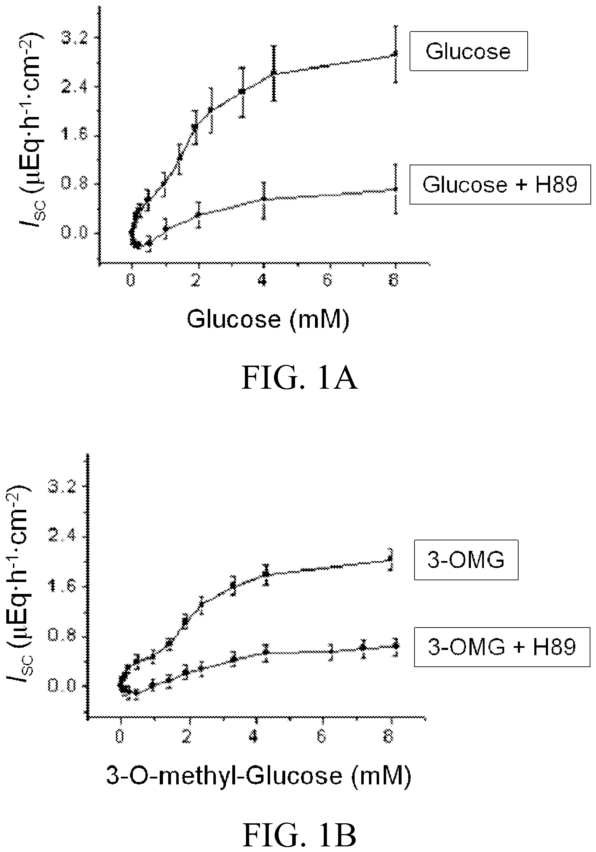

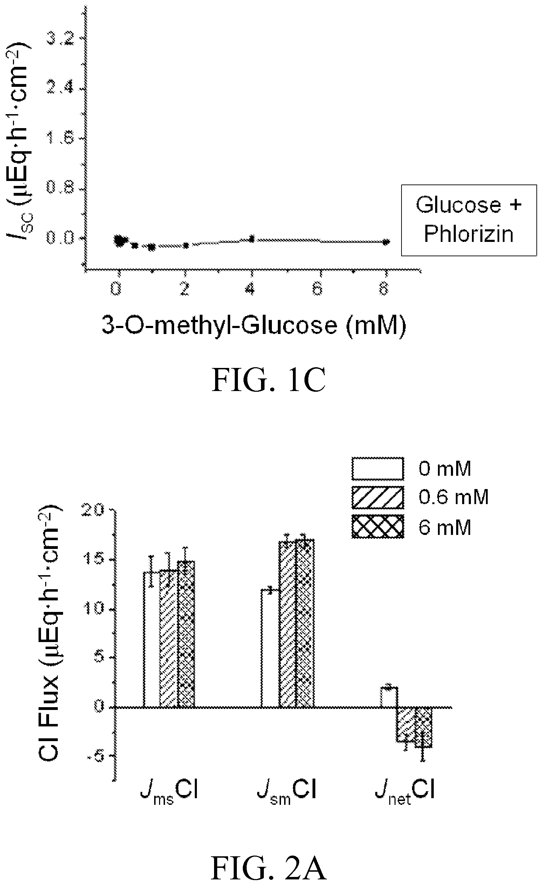

**Figure 1** shows the saturation kinetics for Na⁺-coupled glucose and Na⁺-coupled 3-O-methylglucose (3-OMG) transport. **(A)** Increasing concentration of lumen glucose results in a concentration-dependent increase in *I*_{sc}. Nonlinear curve fit with the Michaelis-Menten model for enzyme kinetics shows Vₘₐₓ = 3.3 ± 0.19 µeq·h⁻¹·cm⁻² and Kₘ = 0.24 ± 0.06 mM. **(B)** Increasing lumen concentration of 3-OMG results in a concentration-dependent increase in *I*_{sc} with a Vₘₐₓ = 1.9 ± 0.13 µeq·h⁻¹·cm⁻² and Kₘ = 0.22 ± 0.07 mM. Increasing concentration of 3-OMG in tissues pre-treated with H-89 results in a significant decrease in *I*_{sc}, when compared to that of tissues not pre-treated with H-89. **(C)** Addition of increasing concentrations of 3-OMG in tissues pre-treated with phlorizin showed no response to glucose. The values are obtained from n=6 tissues.

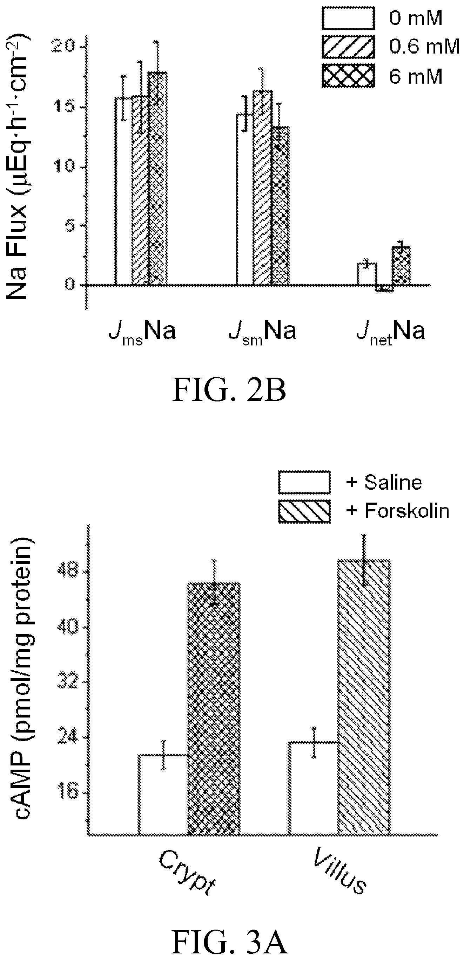

**Figure 2** shows unidirectional and net flux of Na⁺ **(A)** and Cl⁻ **(B). (A)** Incubation of small intestine tissues with glucose at a concentration of 0, 0.6, or 6 mM results in no significant difference in *J*ₘₛCl⁻. Glucose induces an increase in *J*ₛₘCl⁻ in the small intestine. Specifically, *J*ₛₘCl⁻ is significantly higher in the presence of 0.6 and 6 mM glucose, when compared to that of 0 mM glucose. At 0 mM glucose, significant Cl⁻ absorption is observed (when compared to Cl⁻ absorption level at 0.6 mM and 6 mM glucose), while at 0.6 mM and 6 mM glucose, Cl⁻ secretion is observed. **(B).** At 0 mM glucose, net Na⁺ absorption is observed in small intestine tissues. Minimal Na⁺ absorption is observed at 0.6 mM glucose, whereas significant Na⁺ absorption is observed at 6 mM glucose. Unidirectional fluxes (*J*ₘₛ and *J*ₛₘ) do not show a significant difference at 0, 0.6 or 6mM glucose. The values are obtained from n=8 tissues.

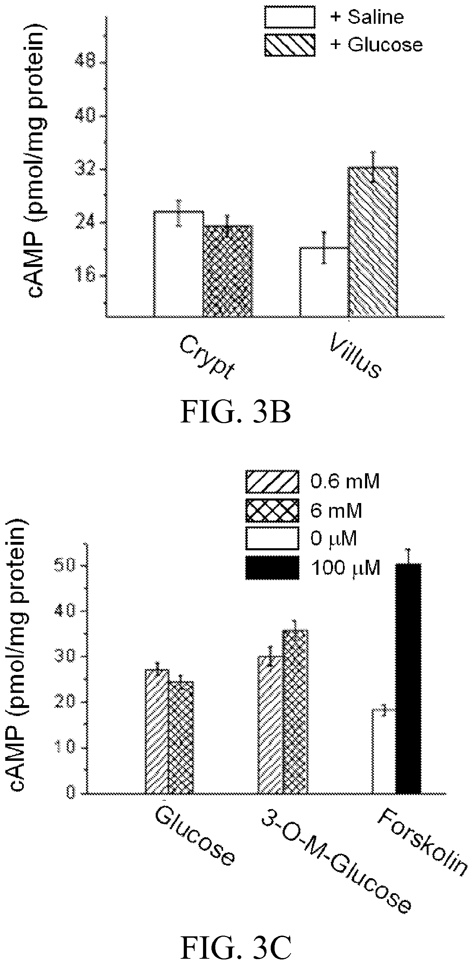

**Figure 3** shows effects of glucose and 3-O-methyl-glucose on intracellular cAMP levels in villus, crypt and whole cell fraction of ileum. **(A)** Forskolin treatment significantly increases intracellular cAMP levels in crypt and villus cells in a similar manner. **(B)** Incubation of cells with 8 mM glucose results in a significant increase in the intracellular cAMP levels in villus cells, but not in crypt cells. **(C)** Incubation of the mucosal scraping consisting of both the villus and the crypt epithelial cells with glucose and 3-O-methyl-glucose, respectively, results in a significant increase in intracellular cAMP levels. Incubation of cells with 3-O-methyl-glucose at 6 mM results in a small but significant increase in intracellular cAMP levels. Incubation of cells with different concentrations of glucose produces similar effects on intracellular cAMP levels. Columns represent the mean values and bars show the S.E.M. The values are obtained from n=4 different mice repeated in triplicate. cAMP levels are standardized to protein levels from respective fractions and expressed as pmol (mg protein)⁻¹. * *P* < 0.001 compared with group after addition of forskolin or glucose; #P < 0.01 comparison between saline treated and glucose treated villus cells. NS = not significant (Bonferroni's multiple comparisons).

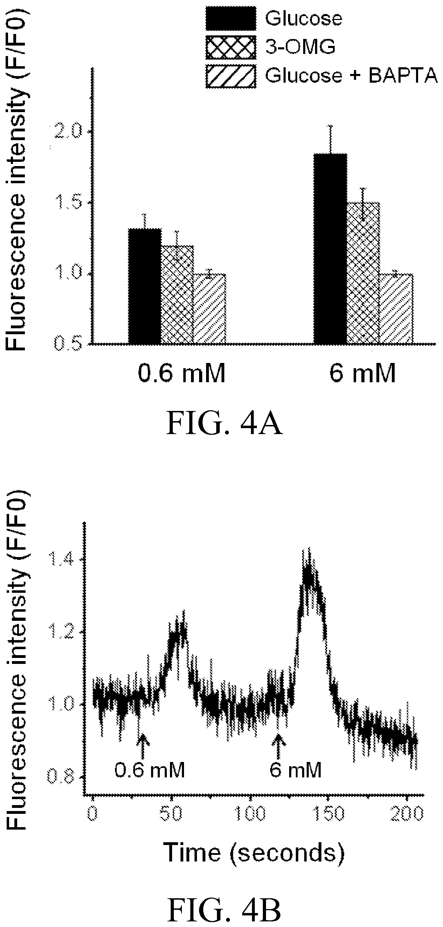

**Figure 4** shows effects of glucose and 3-O-methyl-glucose on intracellular Ca²⁺ levels in Caco-2 cells. **(A)** Incubation of Caco-2 cells with 0.6 mM glucose results in an increase in fluorescence, when compared to control. Incubation with 6 mM glucose results in a significant increase in fluorescence, when compared to that of control and 0.6 mM glucose. In cells pre-incubated (for a period of 45 minutes) with 1,2-**b**is(o-**a**mino**p**henoxy)ethane-N,N,N',N'-tetraacetic acid) (BAPTA-AM), glucose fails to stimulate any increase in intracellular Ca²⁺ level. Incubation with 3-OMG results in a significantly lower glucose-stimulated increase in intracellular Ca²⁺ levels than that of glucose at similar concentrations. **(B)** Representative trace showing increase in intracellular Ca²⁺ levels stimulated by glucose at a concentration of 0.6mM and 6 mM.

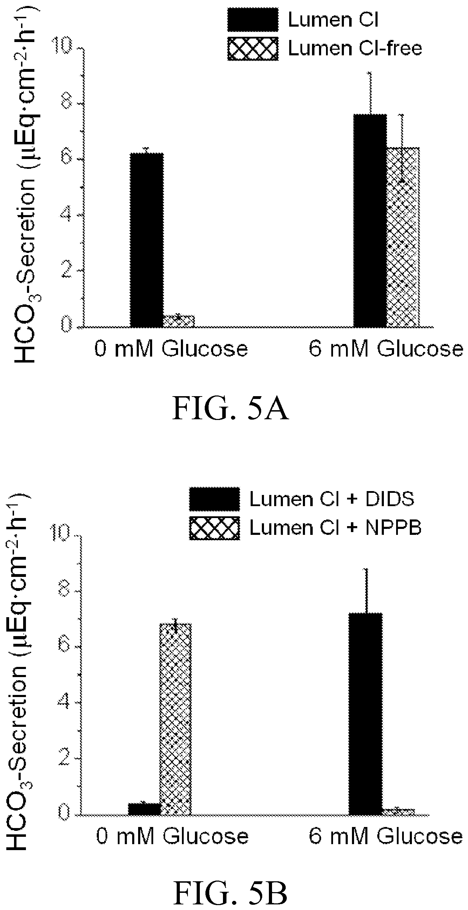

**Figure 5** shows results of pH stat experiments showing Cl⁻-dependent and Cl⁻-independent HCO₃⁻ secretion. **(A)** In the absence of glucose, there is a minimal level of Cl⁻-independent HCO₃⁻ secretion. In the presence of 6 mM glucose, removal of lumen Cl⁻ does not result in a significant decrease in HCO₃⁻ secretion. **(B)** Effect of anion exchange inhibitor and anion channel blocker on HCO₃⁻ secretion. Experiments are performed in the presence of lumen Cl⁻. In the absence of glucose, addition of 100 µM 4,4'-diisothiocyano-2,2'-stilbenedisulfonic acid (DIDS) abolishes HCO₃⁻ secretion while 10 µM 5-nitro-2-(3-phenylpropylamino)-benzoic acid (NPPB) does not have any inhibitory effect on HCO₃⁻ secretion. In the presence of 6 mM glucose, NPPB, but not DIDS, inhibits HCO₃⁻ secretion. The values are obtained from n = 6 tissues from different mice. P < 0.001.

### DETAILED DISCLOSURE

The present invention provides therapeutic compositions for use in methods for treating diarrhea, for providing rehydration, for correcting electrolyte and fluid imbalances, and/or for improving small intestine function.

In one embodiment, the present disclosure provides a composition formulated for enteral administration, wherein the composition does not contain glucose. In a preferred embodiment, the composition is formulated as an oral rehydration drink (ORD). In another preferred embodiment, the composition is in a powder form, and can be reconstituted in water for use as an ORD.

In one embodiment, the composition of the present disclosure comprises one or more ingredients selected from free amino acids; electrolytes; di-peptides and/or oligo-peptides; vitamins; and optionally, water, therapeutically acceptable carriers, excipients, buffering agents, flavoring agents, colorants, and/or preservatives. In one embodiment, the total osmolarity of the composition is from about 100 mosm to 250 mosm. In one embodiment, the composition has a pH from about 2.9 to 7.3. In one embodiment, the present disclosure provides a treatment comprising administering, via an enteral route, to a subject in need of such treatment, an effective amount of a composition of the disclosure. The composition can be administered once or multiple times each day. In a preferred embodiment, the composition is administered orally.

In a preferred embodiment, the present invention provides treatment of diarrhea induced by rotavirus infection and/or NSP4. In another preferred embodiment, the present disclosure results in decreased Cl⁻ and/or HCO₃⁻ secretion and/or improved fluid absorption.

### Induction of Anion Secretion by Glucose

In accordance with the present disclosure, it has been found that lumen glucose induces net ion secretion in the small intestine. Specifically, glucose induces an active chloride secretion mediated by increased intracellular cAMP and Ca²⁺ levels. Also, net Na⁺ transport in the small intestine is absorptive at high glucose concentrations. In addition, glucose results in bicarbonate secretion in the small intestine.

The present inventors have shown that an increase in intracellular cAMP level mediates Cl⁻ and/or HCO₃⁻ secretion. The Cl⁻ and/or HCO₃⁻ secretion is largely mediated by cystic fibrosis transmembrane conductance regulator (CFTR) ion channels, which have numerous (∼ 20) potential serine and threonine phosphorylation sites. Protein kinase A (PKA) and protein kinase C (PKC) are known to activate CFTR anion channels. In patch clamp studies, it has been shown that CFTR channels are inactivated ("run down") quickly unless continuously activated by PKA, signifying the importance of PKA in the activation of CFTR. Consistent with this observation, pre-treatment of small intestine cells with a potent PKA inhibitor H89 results in a significant reduction in glucose-stimulated net increase in *Isc.*

PKA antagonists have been shown to inhibit SGLT1 protein expression following glucose exposure (Dyer et al. (2003) Eur. J. Biochem. 270(16):3377-3388). CFTR channels are activated by the cAMP-dependent protein kinase (PKA), leading to anion secretion. Glucose-stimulated increase in *I*_{sc} in the small intestine is partially mediated by CFTR-mediated ion transport.

Glucose as well as PKA agonists (such as cAMP) have been shown to increase the trafficking of SGLT1 to the brush border membrane (Wright et al. (1997) J. Exp. Biol. 200(Pt 2):287-293; Dyer et al. (2003) Eur. J. Biochem. 270(16):3377-3388). The decrease in Vmax indicates a total decrease in current, which represents a decrease in glucose transport. The decrease in Vmax could result from a reduction of the total number of glucose transporter SGTL1, which is mostly found villus epithelial cells. The loss of villus results in a significant loss of available transporter for taking glucose into the cells.

It has been found that incubating enterocytes with glucose increases intracellular cAMP levels. A greater increase in glucose-induced intracellular cAMP level is observed in villus cells than in crypt cells. Incubating enterocytes with forskolin increases intracellular cAMP levels in both crypt and villus cells (Fig 3A). SGLT1-mediated glucose transport occurs primarily in villus cells instead of in crypt cells, as a greater number of SGLT-1 are located in the villus region than in the crypt region (Knickelbein et al. (1988) J. Clin. Invest. 82(6):2158-2163). Accordingly, increasing glucose concentrations in crypt cells does not result in increased cAMP response (Fig 3B).

Even at low concentration (e.g., 0.6 mM glucose that is approximately half of its Vₘₐₓ), lumen glucose induces net anion secretion. At higher concentrations of glucose, sodium absorption is predominant. Increased lumen glucose concentration increases intracellular cAMP and Ca²⁺ levels. Previous studies have shown that Kₘ for Na⁺-coupled glucose transport is in a range of 0.2 to 0.7 mM (Lo & Silverman (1998) J. Biol. Chem. 273(45):29341-29351).

The presence of a residual glucose-mediated increase in *Isc* in cells pre-treated with H-89 indicates that PKA independent pathway(s) exist in glucose-induced anion secretion. Electrogenic anion secretion across the small intestine is mediated by ion channels, which can be classified based on their mechanisms of activation, such as activation by cAMP, Ca²⁺, cell-volume and membrane potential.

It has also been found that lumen glucose induces an increase in intracellular Ca²⁺ levels. Also, the glucose-induced Cl⁻ secretion is mediated by PKA-dependent as well as PKA-independent pathways. This indicates that, in addition to CFTR, calcium activated chloride channels (CaCCs) also play a role in glucose-induced anion secretion.

In addition, glucose stimulates electrogenic HCO₃⁻ secretion. Small intestine cells incubated with glucose exhibit higher levels of HCO₃⁻ secretion in lumen Cl⁻-containing solution than in lumen Cl⁻ free solution (Fig 4A & 4B). These results indicate that anion channels mediate HCO₃⁻ secretion in the presence of glucose. Also, addition of glucose results in a slight decrease in Cl⁻-HCO₃⁻ exchange, when compared to cells with no glucose addition. This decrease may be secondary to an increase in intracellular cAMP level with glucose. This also indicates that glucose induces anion channel-mediated secretion and inhibits electroneutral Cl⁻-HCO₃⁻ exchange.

In addition, small intestine cells were incubated with an anion channel blocker (100 mM NPPB) and an anion exchange inhibitor (100 mM DIDS), respectively. There was significant inhibition of glucose-induced, anion channel-mediated HCO₃⁻ secretion by NPPB (100 mM) (4.2 ± 0.7 vs 7.6 ± 1.5 mEq.h⁻¹.cm⁻²).

In the presence of anion channel inhibitors, residual HCO₃⁻ secretion is still observed. This indicates that Cl⁻-HCO₃⁻ exchange is present in glucose-mediated secretion. This also indicates that an elevated intracellular calcium level could inhibit sodium-hydrogen exchanger 3 (NHE3) activity during normal digestive function as well as in certain disease conditions. This also indicates that SGLT1 plays a dual role in regulating sodium absorption and, at some time, stimulating a secretory and/or an absorptive defect.

The discovery of glucose-induced secretory mechanism can be used in the treatment of gastrointestinal diseases including diarrhea. Patients with acute diarrheal diseases commonly have impaired glucose absorption that occurs in the upper gastrointestinal tract. The presence of unabsorbed carbohydrates can exert an osmotic effect in the bowel, leading to diarrhea. In addition, glucose increases intracellular Ca²⁺ and/or cAMP levels and induces anion secretion. The secretory effects of glucose have been previously understudied or masked by concurrent Na⁺-glucose absorption. Also, due to its secretory effects, glucose administration particularly exacerbates gastrointestinal diseases with impaired Na⁺-glucose absorption, such as Crohn's disease and irradiation or chemotherapy-induced enteritis that are associated with shortening of the villi and, therefore, extremely compromised absorption.

During rotavirus infection, although there is a predominant glucose-coupled Na⁺ absorption via the sodium-dependent glucose cotransporter (SGLT-1) that is primarily expressed in villus cells, there is a significant calcium activated Cl⁻ secretion via the calcium activated chloride channel (CaCC or TMEM-16a) in the small intestine. In addition, intracellular glucose activates calcium-activated chloride and fluid secretion. Non-structural protein (NSP4) is an entero-toxin produced by rotavirus. It is discovered that glucose and NSP4, when administered together, results in sustained chloride secretion in cells. As a result, the existing ORD formulations that contain a significant amount of glucose further increase the calcium-stimulated chloride secretion, thereby worsening rotavirus-induced diarrhea.

### Therapeutic Compositions

In one aspect, the present invention provides therapeutic compositions for use in methods for treating diarrhea, for providing rehydration, for correcting electrolyte and fluid imbalances, and/or for improving small intestine function.

In one embodiment of the disclosure, the composition is formulated for enteral administration and does not contain glucose. In a preferred embodiment, the composition is formulated as an oral rehydration drink. In another preferred embodiment, the composition is in a powder form, and can be reconstituted in water for use as an oral rehydration drink.

In a further embodiment, the composition does not contain any substrate of glucose transporters. In a further specific embodiment, the composition does not contain agonists of sodium-dependent glucose cotransporter (SGLT-1) including, but not limited to, glucose analogs (e.g., non-metabolizable glucose agonists for SGLT-1) and other carbohydrates (such as sugars).

Various substrates of SGLT-1 are known in the art including, but not limited to, non-metabolizable glucose analogs such as α-methyl-D-glucopyranoside (AMG), 3-O-methylglucose (3-OMG), deoxy-D-glucose, and α-methyl-D-glucose; and galactose. Substrates of glucose transporters (e.g., SGLT-1) can be selected based on agonist assays as is known in the art. Also, structural modifications of the glucose and other carbohydrates (such as sugars) can be made to obtain substrates of glucose transporters (e.g., SGLT-1).

In one embodiment, the composition does not contain glucose. In a further embodiment, the composition does not contain carbohydrates (such as di-, oligo-, or polysaccharides) or other compounds that can be hydrolyzed into glucose or a substrate of glucose transporters (*e.g*., SGLT-1).

In one embodiment of the disclosure, the composition comprises, consists essentially of, or consists of, one or more ingredients selected from free amino acids; electrolytes; dipeptides and/or oligo-peptides; vitamins; and optionally, water, therapeutically acceptable carriers, excipients, buffering agents, flavoring agents, colorants, and/or preservatives.

In another alternative embodiment of the disclosure, the composition comprises, consists essentially of, or consists of, one or more ingredients selected from free amino acids; electrolytes; di-peptides and/or oligo-peptides; vitamins; and, optionally, water, therapeutically acceptable carriers, excipients, buffering agents, flavoring agents, colorants, and/or preservatives;
wherein glucose transporters (*e.g*., SGLT-1) substrates (such as, glucose, glucose analogs) and/or compounds (such as carbohydrates) that can be hydrolyzed into a substrate of glucose transporters (*e.g*., SGLT-1), if present in the composition, are present in a total concentration of lower than 0.05 mM or any concentration lower than 0.05 mM including, but not limited to, lower than 0.04, 0.03, 0.02, 0.01, 0.008, 0.005, 0.003, 0.001, 0.0005, 0.0003, 0.0001, 10⁻⁵, 10⁻⁶, or 10⁻⁷ mM. In one embodiment, the anti-diarrhea composition does not contain sugar. In another embodiment, the anti-diarrhea composition does not contain glucose transporters (*e.g*., SGLT-1) substrates (such as, glucose, glucose analogs) and/or compounds (such as carbohydrate) that can be hydrolyzed into a substrate of glucose transporters (*e.g*., SGLT-1).

Amino acids useful for the anti-diarrhea composition of the disclosure include, but are not limited to, alanine, asparagine, aspartic acid, cysteine, aspartic acid, glutamic acid, phenylalanine, glycine, histidine, isoleucine, lysine, leucine, methionine, proline, glutamine, arginine, serine, threonine, valine, tryptophan, and tyrosine.

In one embodiment, the subject disclosure provides an anti-diarrhea composition, wherein the composition comprises, consists essentially of, or consists of free amino acids lysine, glycine, threonine, valine, tyrosine, aspartic acid, isoleucine, tryptophan, and serine; and optionally, dipeptides or oligopeptides made of one or more of free amino acids selected from lysine, glycine, threonine, valine, tyrosine, aspartic acid, isoleucine, tryptophan, and serine, therapeutically acceptable carriers, electrolytes, buffering agents, preservatives, and flavoring agents.

In one embodiment, the amino acids contained in the anti-diarrhea composition are in the L-form. In one embodiment, the free amino acids contained in the therapeutic composition can be present in neutral or salt forms.

In one embodiment, the therapeutic composition further comprises one or more electrolytes selected from Na⁺, K⁺, Ca²⁺, HCO₃⁻, and Cl⁻. In one embodiment, the therapeutic composition comprises sodium chloride, sodium bicarbonate, calcium chloride, and/or potassium chloride.

In certain embodiments, each free amino acid can be present at a concentration from 4 mM to 40 mM, or any value therebetween, wherein the total osmolarity of the composition is from about 100 mosm to 250 mosm. The term "consisting essentially of," as used herein, limits the scope of the ingredients and steps to the specified materials or steps and those that do not materially affect the basic and novel characteristic(s) of the present invention, e.g., compositions and methods for treatment of gastrointestinal diseases and conditions (which, in certain embodiments, being treatment of diarrhea, such as rotavirus-induced diarrhea), for providing rehydration, for correcting electrolyte and fluid imbalances, and/or for improving small intestine function. For instance, by using "consisting essentially of," the therapeutic composition does not contain any unspecified ingredients including, but not limited to, unspecified free amino acids, di-, oligo-, or polypeptides or proteins; mono-, di-, oligo-, or polysaccharides; or carbohydrates that have a direct beneficial or adverse therapeutic effect on treatment of gastrointestinal diseases and conditions (which, in certain embodiments, being treatment of diarrhea, such as rotavirus-induced diarrhea) for providing rehydration, for correcting electrolyte and fluid imbalances, and/or for improving small intestine function.

Also, by using the term "consisting essentially of," the composition may comprise substances that do not have therapeutic effects on treatment of gastrointestinal diseases and conditions (which, in certain embodiments, being treatment of diarrhea, such as rotavirus-induced diarrhea) for providing rehydration, for correcting electrolyte and fluid imbalances, and/or for improving small intestine function; such ingredients include carriers, excipients, flavoring agents, colorants, and preservatives etc that do not affect treatment of gastrointestinal diseases and conditions (which, in one embodiment, being treatment of diarrhea), for providing rehydration, for correcting electrolyte imbalances, and/or for improving small intestine function.

The term "oligopeptide," as used herein, refers to a peptide consisting of three to twenty amino acids.

The term "oligosaccharide," as used herein, refers to a saccharide consisting of three to twenty monosaccharides. The term "carbohydrates," as used herein, refers to compounds having the general formula of Cₙ(H₂O)ₙ, wherein n is an integer starting from 1; and includes monosaccharaides, disaccharides, oligosaccharides, and polysaccharides.

The total osmolarity of the composition is from about 100 mosm to 250 mosm, or any value therebetween including, but not limited to, 120 mosm to 220 mosm, 150 mosm to 200 mosm, and 130 mosm to 180 mosm.

In another embodiment of the disclosure, the total osmolarity of the composition is from about 230 mosm to 280 mosm, or any value therebetween. In one embodiment of the disclosure, the total osmolarity is from about 250 to 260 mosm. In another embodiment of the disclosure, the composition has a total osmolarity that is any value lower than 280 mosm.

In certain embodiments, the composition has a pH from about 2.9 to 7.3, or any value therebetween including, but not limited to, a pH of 3.3 to 6.5, 3.5 to 5.5, and 4.0 to 5.0.

In certain embodiments, the composition has a pH from about 7.1 to 7.9, or any value therebetween. Preferably, the composition has a pH from about 7.3 to 7.5, more preferably, about 7.2 to 7.4, or more preferably, about 7.2.

In certain embodiments of the disclosure, the composition does not contain one or more ingredients selected from oligo- or polysaccharides or carbohydrates; oligo- or polypeptides or proteins; lipids; small-, medium-, and/or long-chain fatty acids; and/or food containing one or more above-mentioned nutrients.

### Treatment of Gastrointestinal Diseases and Conditions

Another aspect of the present disclosure provides methods for treatment of gastrointestinal diseases and conditions. In certain embodiments, the present invention can be used to treat diarrhea, to provide rehydration, to correct electrolyte and fluid imbalances, and/or to improve small intestine function. In a preferred embodiment, the present invention provides treatment of rotavirus-induced diarrhea. In another preferred embodiment, the present invention provides treatment of diarrhea induced by NSP4.

In one embodiment of the disclosure, the method comprises administering, via an enteral route, to a subject in need of such treatment, an effective amount of a composition of the disclosure. The composition can be administered once or multiple times each day. In one embodiment, the composition is administered orally.

In a preferred embodiment, the present disclosure provides decreased Cl⁻ and/or HCO₃⁻ secretion and/or improved fluid absorption.

The term "treatment" or any grammatical variation thereof (e.g., treat, treating, and treatment *etc*.), as used herein, includes but is not limited to, alleviating or ameliorating a symptom of a disease or condition; and/or reducing the severity of a disease or condition. In certain embodiments, treatment includes one or more of the following: alleviating or ameliorating diarrhea, reducing the severity of diarrhea, reducing the duration of diarrhea, promoting intestinal healing, providing rehydration, correcting electrolyte imbalances, improving small intestine mucosal healing, and increasing villus height in a subject having diarrhea.

The term "effective amount," as used herein, refers to an amount that is capable of treating or ameliorating a disease or condition or otherwise capable of producing an intended therapeutic effect.

The term "subject" or "patient," as used herein, describes an organism, including mammals such as primates, to which treatment with the compositions according to the present invention can be provided. Mammalian species that can benefit from the disclosed methods of treatment include, but are not limited to, apes, chimpanzees, orangutans, humans, monkeys; domesticated animals such as dogs, cats; live stocks such as horses, cattle, pigs, sheep, goats, chickens; and other animals such as mice, rats, guinea pigs, and hamsters.

In one embodiment, the human subject is an infant of less than one year old, or of any age younger than one year old, such as 10 months old, 6 months old, and 4 months old. In another embodiment, the human subject is a child of less than five years old, or of any age younger than five years old, such as four years old, three years old, and two years old. In one embodiment of the disclosure, the subject in need of treatment of the present disclosure is suffering from diarrhea.

The present invention can be used to treat diarrhea. In certain embodiments, the present invention can be used to treat diarrhea caused by pathogenic infections including, but not limited to, infections by viruses, including, but not limited to, rotavirus, Norwalk virus, cytomegalovirus, and hepatitis; bacteria including, but not limited to, campylobacter, salmonella, shigella, *Vibrio cholerae,* and *Escherichia coli;* parasites including, but not limited to, *Giardia lamblia* and cryptosporidium. In a preferred embodiment, the present invention can be used to treat rotavirus-induced diarrhea.

In certain embodiments, the present invention can be used to treat diarrhea caused by injury to the small intestine caused by, for example, infection, toxins, chemicals, alcohol, inflammation, autoimmune diseases, cancer, chemo-, radiation, proton therapy, and gastrointestinal surgery.

In certain embodiments, the present invention can be used in the treatment of diarrhea caused by diseases including, but not limited to, inflammatory bowel diseases (IBD) including Crohn's disease and ulcerative colitis; irritable bowel syndrome (IBS); autoimmune enteropathy; enterocolitis; and celiac diseases.

In certain embodiments, the present invention can be used in the treatment of diarrhea caused by gastrointestinal surgery; gastrointestinal resection; small intestinal transplant; post-surgical trauma; and radiation-, chemo-, and proton therpy-induced enteritis.

In another embodiment, the present invention can be used to treat alcohol-related diarrhea. In another embodiment, the present invention can be used to treat traveler's diarrhea and/or diarrhea caused by food poisoning.

In certain embodiments, the present invention can be used in the treatment of diarrhea caused by injury to the small intestine mucosa, for example, diarrheal conditions in which there is a reduced villous height, decreased mucosal surface areas in the small intestine, and villus atrophy, e.g., partial or complete wasting away of the villous region and brush border. In certain embodiments, the present invention can be used in the treatment of diarrhea caused by injury to small intestine mucosal epithelial cells, including the mucosa layer of duodenum, jejunum, and ileum.

In one embodiment, the present invention can be used to treat secretory diarrhea. In certain embodiments, the present invention can be used to treat secretory diarrhea mediated via the CFTR channels and/or CaCC channels (e.g., TMEM-16a). In one embodiment, the present invention can be used to treat acute and/or chronic diarrhea.

In one embodiment, the present invention can be used to treat diarrhea caused by malabsorption of nutrients. In one embodiment, the present invention can be used to treat secretory diarrhea caused by reduced level or functional activity of glucose transporters such as SGLT-1.

As used herein, the term "diarrhea" refers to a condition in which three or more unformed, loose or watery stools occur within a 24-hour period. "Acute diarrhea" refers to diarrheal conditions that last no more than four weeks. "Chronic diarrhea" refers to diarrheal conditions that last more than four weeks.

In one embodiment, the present disclosure does not involve the administration of one or more of the following ingredients selected from glucose, glucose analogs, substrates of glucose transporters (e.g., SGLT-1), di-, oligo-, or polysaccharides; carbohydrates; or molecules that can be hydrolyzed into glucose or a substrate of glucose transporters (e.g., SGLT-1).

In certain alternative embodiments, the present disclosure comprises administering one or more ingredients selected from glucose; glucose analogs; substrates of glucose transporters (*e.g*., SGLT-1); di-, oligo-, or polysaccharides; carbohydrates; or molecules that can be hydrolyzed into glucose or a substrate of glucose transporters (*e.g*., SGLT-1), wherein the total concentration of these ingredients is lower than 0.05 mM or any concentration lower than 0.05 mM including, but not limited to, lower than 0.04, 0.03, 0.02, 0.01, 0.008, 0.005, 0.003, 0.001, 0.0005, 0.0003, 0.0001, 10⁻⁵, 10⁻⁶, or 10⁻⁷ mM.

### Formulations and Administration

The present disclosure provides for therapeutic or pharmaceutical compositions comprising a therapeutically effective amount of the subject composition and, optionally, a pharmaceutically acceptable carrier. Such pharmaceutical carriers can be sterile liquids, such as water. The therapeutic composition can also comprise excipients, flavoring agents, colorants, and preservatives etc that do not affect treatment of gastrointestinal diseases and conditions (which, in one embodiment, being treatment of diarrhea), for providing rehydration, for correcting electrolyte and fluid imbalances, and/or for improving small intestine function.

In an embodiment, the therapeutic composition and all ingredients contained therein are sterile. In certain preferred embodiments, the composition is formulated as a drink, or the composition is in a powder form and can be reconstituted in water for use as a drink.

The term "carrier" refers to a diluent, adjuvant, excipient, or vehicle with which the compound is administered. Examples of suitable pharmaceutical carriers are described in "Remington's Pharmaceutical Sciences" by E. W. Martin. Such compositions contain a therapeutically effective amount of the therapeutic composition, together with a suitable amount of carrier so as to provide the form for proper administration to the patient. The formulation should suit the enteral mode of administration.

The invention also provides a pharmaceutical pack or kit as specified in the claims comprising one or more containers filled with one or more of the ingredients, *e.g*., compound, carrier, or the pharmaceutical compositions of the invention. The ingredients of the composition can be packaged separately or can be mixed together. The kit can further comprise instructions for administering the composition to a patient.

### Materials and Methods

### Animal preparation

Normally fed, 8-week-old, male NIH Swiss mice are sacrificed by CO₂ inhalation, followed by cervical dislocation. The small intestine is gently removed, and the segment is washed and flushed in ice-cold Ringer's solution. Then the mucosa is separated from the serosa and the muscular layers by striping through the submucosal plane as previously described (Zhang et al. (2007) J Physiol 581(3):1221-1233). Following exsanguinations, ileal mucosa is obtained from a 10 cm segment close to the caecum. All experiments are approved by the University of Florida Institutional Animal Care and Use Committee.

### Bio-electric measurements

Ion transport studies are performed on ileal sheets. Tissues are then mounted in between the two halves of an Ussing type-Lucite chamber with 0.3cm² exposed surface areas (P2304, Physiologic Instruments, San Diego, CA, USA). Regular Ringer's solution (115mM NaCl, 25mM NaHCO₃, 4.8mM K₂HPO₄, 2.4mM KH₂PO₄, 1.2mM MgCl₂ and 1.2mM CaCl₂) bubbled with 95% O₂: 5%CO₂ is used bilaterally as bathing solution for the tissues and the temperature is maintained constant at 37°C. The chambers are balanced to eliminate osmotic and hydrostatic forces. Resistance due to fluid is also compensated. The tissues are allowed to stabilize. The basal short-circuit current (I_{sc}) and the corresponding conductance (G) are recorded using a computer controlled voltage/current clamp device (VCC MC-8, Physiologic Instruments).

### Flux studies

Isotope of Sodium, ²²Na, is used to study Na flux across the mucosa under basal conditions followed by addition of glucose. Conductance-paired tissues are designated to study serosal to mucosal flux (Jₛₘ) representing secretory function, and mucosal to serosal flux (Jₘₛ) representing absorptive function. ²²Na is added in to the designated side of the tissue and 500µl samples are collected every 15 minutes from the other side. In a separate set of tissues ³⁶Cl is added to either the serosal or the mucosal side. Glucose of 8mM concentration is added into the chamber for full stimulation, and the corresponding changes in I_{sc} and conductance are recorded. Conductance is recorded based on the Ohm's law.

Three samples are collected under each condition. Radioactvity is counted using gamma counter. Tissues with conductance less than 10% change are matched and the average Jₙₑₜ = Jₘₛ - Jₛₘ is calculated.

### Protein Kinase A (PKA) inhibitor studies

Tissues paired with similar conductance and current are treated with or without 100µM H-89 (Santa Cruz Biotechnology, Inc, Santa Cruz, CA), an irreversible protein kinase A (PKA) inhibitor. The tissues are incubated with H-89 for 30 minutes. Increasing concentrations of glucose (0.015 - 8mM) are added every 5 minutes and the peak current is noted. Saturation kinetic constant is calculated for the corresponding Kₘ and Vₘₐₓ for treated and untreated tissues.

### Caco-2 Cell Culture

Caco-2 cells differentiate post-confluence into cells with functional characteristics of fetal ileal epithelium. Caco-2 cells produce microvilli and have increased expression of small intestine specific transport proteins including SGLT1 and are therefore widely used as a model system for studying enterocyte function.

Caco-2 cells are obtained from ATTC and cultured in Dulbecoo's modified Eagle's medium supplemented with 10% fetal calf serum (FCS) and 1% nonessential amino acids at 37°C and 5% CO₂. Caco-2 cells are passaged for 20-25 times and are seeded (2 x 10⁵ cells/dish) on 5 cm petri-dishes and grown until 80% confluence, when the FCS concentration is changed to 5%. Cells are grown for another 10 days before they are used for functional studies.

### Confocal Ca²⁺ fluorescence microscopy

Caco2 cells grown in 25 mm round coverslips are mounted on the bath chamber RC-21BR attached to series 20 stage adapter (Warner Instruments, CT USA). The cells are maintained at 37°C using a single channel table top heater controller (TC-324B, Warner Instruments, CT USA). Cells are loaded with the fluorescent calcium indicator Fluo-8 AM dye (Cat # 0203, TEFLab, Inc., Austin, TX USA) at 0.5 µM concentration at room temperature and incubated for 45 minutes. Confocal laser scanning microscopy is performed using an inverted Fluoview 1000 IX81 microscope (Olympus, Tokyo, Japan) and a U Plan S-Apo 20× objective. Fluorescence is recorded by argon lasers with excitation at 488 nm and emission at 515 nm. The Fluorescent images are collected with scanning confocal microscope. Solutions of either Ringer, glucose-containing Ringer's or BAPTA-AM-containing glucose-Ringer's solution are added to the bath using a multi-valve perfusion system (VC-8, Warner instruments, Hamden CT, USA) controlled using a VC-8 valve controller (Warner instruments, Hamden CT, USA). Changes are recorded and fluorescence is measured for various cells. Cells are washed with Ringer's solution and the experiment is repeated with the use of 3-O-methylglucose and carbechol (positive control).

### Colorimetric cAMP measurements

Freshly isolated mucosal scrapings of ileal epithelial cells are washed three times in Ringer's solution containing 1.2 mM Ca²⁺ at 37°C. Washed cells are then divided into two groups and treated with either saline or 6 mM glucose and incubated for 45 minutes. Cells are treated with 0.1 M HCl to stop endogenous phosphodiesterase activity. The lysates are then used for cAMP assay using cAMP direct immunoassay kit (Calbiochem, USA).

The quantitative assay of cAMP uses a polyclonal antibody to cAMP that binds to cAMP in samples in a competitive manner. After a simultaneous incubation at room temperature, the excess reagents are washed away and substrates are added. After a short incubation time, the reaction is stopped and the yellow color generated is read at 405 nm. The intensity of the color is inversely proportional to the concentration of cAMP in standards and samples. cAMP levels are standardized to protein levels from respective fractions and expressed in pmol (mg protein)⁻¹.

Forskolin treated cells are used as a positive control. Glucose and forskolin treated cells are incubated for 45 minutes. All the assays are performed in triplicate and repeated until n = 4 different mice.

### EXAMPLES

Following are examples which illustrate procedures and embodiments for practicing the invention. All percentages are by weight and all solvent mixture proportions are by volume unless otherwise noted.

### EXAMPLE 1 - GLUCOSE-STIMULATED INCREASE IN Isc IN ILEUM

This Example shows that glucose stimulates an increase in *I_{sc}* in mouse ileum. Specifically, addition of glucose (8 mM) to the lumen side results in a significant increase in *I*_{sc} when compared to its basal level (3.4 ± 0.2 vs 1.1 ± 0.1 µEq.h⁻¹.cm⁻²). The *I*_{sc} obtained using standard Ussing chamber studies is a summation of net ion movement across the epithelium (*I*_{sc} = *J*ₙₑₜNa⁺ + *J*ₙₑₜCl⁻ + *J*ₙₑₜ HCO₃⁻ - *J*ₙₑₜK⁺).

There are no known Na⁺ absorptive (ENaC-mediated) or Na⁺ secretory mechanisms in the small intestine. Treatment of the mucosal side of the small intestine with 10 µM amiloride, an epithelial sodium channel inhibitor, produces no effect on *I*_{sc}.

Therefore, the basal *I*_{sc} of 1.1 ± 0.1 µEq.h⁻¹.cm⁻² is primarily due to cystic fibrosis transmembrane conductance regulator (CFTR) activity from the crypt and K⁺ secretory current.

To determine the saturation kinetics of Na⁺-coupled glucose transport, increasing concentrations of glucose up to 8 mM are added to the lumen side in the presence of 140 mM Na⁺. Increasing concentrations of glucose results in an enhanced but saturable rate of *I*_{sc} (Fig. 1A), with a *K*ₘ of 0.24 ± 0.03 mM and a *V*ₘₐₓ of 3.6 ± 0.19 µeq·h⁻¹·cm⁻² for glucose. At glucose concentrations ranging from 0.5 to 0.7 mM, the glucose saturation kinetics show early signs of saturation; nevertheless, continued increase in glucose concentrations results in continued increase in *I*_{sc}, thereby yielding a knick in the glucose saturation curve at glucose concentrations of 0.5 to 0.7 mM.

### EXAMPLE 2 - 3-O-METHYL-GLUCOSE-STIMULATED INCREASE IN Isc

This Example investigates whether the glucose saturation kinetics observed in Example 1 are due to SGLT1-mediated transport but not due to glucose metabolism in the epithelial cells. Specifically, 3-O-methyl-glucose (3-OMG), a poorly metabolized form of glucose, is added to the lumen side to study saturation kinetics of Na⁺-coupled glucose transport.

Figure 1B shows the saturation kinetics of 3-OMG, with a Vₘₐₓ of 2.3 ± 0.13 µeq·h⁻¹·cm⁻² and a Kₘ of 0.22 ± 0.07 mM). Addition of 3-OMG results in a significant decrease in Vₘₐₓ (2.3 ± 0.13 µeq·h⁻¹·cm⁻² vs 3.4 ± 0.2 µeq·h⁻¹·cm⁻²) with no change in Kₘ in the Na⁺-coupled glucose transport, when compared to that with glucose. Similar to glucose, a knick is observed with 3-OMG at concentrations 0.5 to 0.7 mM (Fig. 1B).

### EXAMPLE 3 - GLUCOSE-STIMULATED Isc IN THE PRESENCE OF H-89

Based on the currently-known transport mechanisms, the glucose-stimulated increase in *I*_{sc} could result from electrogenic anion secretion or electrogenic Na⁺ absorption.

Protein Kinase A (PKA), also known as the cAMP-dependent protein kinase, is required in the activation of CFTR channels. To study the role for PKA in glucose-induced increase in *I*_{sc}, tissues are mounted in Ussing chambers and incubated with H-89, a PKA inhibitor, for 45 minutes. Subsequently, the tissues are used for studying glucose saturation kinetics.

In the presence of H-89, glucose shows a Vₘₐₓ of 0. 8 ± 0.06 µEq.cm⁻².h⁻¹ and a Kₘ of 0.58 ± 0.08 mM. The knick in the glucose saturation curve (observed when ileal tissues are incubated with glucose at concentrations ranging from 0.5 to 0.7 mM) disappears altogether when ileal cells are pre-treated with H-89, with a shift of the saturation curve to the right (Fig. 1C). The results indicate the inhibition of PKA-dependent transport processes at low concentrations of glucose.

Similar to the glucose saturation curve, 3-OMG also shows a PKA-sensitive current. The 3-OMG saturation curve (with H-89 incubation) is not significantly different from that observed with glucose (with H-89 incubation) (Fig 1A & B).

**Table 1 Changes in glucose and 3-O-methly-glucose saturation kinetics in the presence and absence of H-89 - a PKA inhibitor.**

| | Vₘₐₓ | Kₘ | Vₘₐₓ | Kₘ |
|---|---|---|---|---|
| PKA Inhibitors | - | - | H-89 | H-89 |
| Glucose | 3.6 ± 0.2 | 0.2 ± 0.1 | 1.6 ± 0.1 | 0.5 ± 0.1 |
| 3-OMG | 2.7 ± 0.1 | 0.2 ± 0.1 | 1.4 ± 0.1 | 0.6 ± 0.1 |

| | | | | |
|---|---|---|---|---|
| * Part of glucose and 3-OMG-stimulated current is abolished in the presence of PKA. Results are from n= 8 tissues. | | | | |

The results indicate that the PKA-inhibitable current (shown in Table 1) results from the Na⁺-coupled glucose transport, instead of from other intracellular metabolisms involving glucose (Table 1).

PKA plays a significant role in cAMP-mediated anion secretion and SGLT1-mediated Na⁺ and glucose absorption. The presence of H-89-insensitive current indicates that glucose stimulates non-PKA-mediated anion secretion (such as intracellular Ca²⁺-mediated secretion).

### EXAMPLE 4 - ABOLISHMENT OF GLUCOSE-STIMULATED INCREASE IN Isc IN THE PRESENCE OF PHLORIZIN

To investigate whether inhibition of glucose transport abolishes PKA-sensitive current, experiments are conducted using phlorizin (Santa Cruz Biotechnology, Inc, Santa Cruz, CA, USA), a reversible competitive inhibitor of SGLT1. Specifically, ileal tissues mounted in Ussing chamber are treated with 100 µM phlorizin on the lumen side and glucose saturation kinetic studies are conducted.

The results show that glucose-stimulated and /or 3-OMG increase in *I*_{sc} is completely abolished in the presence of phlorizin (Fig 1C). The results indicate that glucose transporter activity via SGLT1 is essential for the PKA-sensitive and insensitive current.

### EXAMPLE 5 - EFFECT OF GLUCOSE ON UNIDIRECTIONAL AND NET FLUX OF SODIUM

Isotopic flux measurements of Na⁺ are performed using ²²Na at a steady-state rate of transfer from either mucosa to serosa *J*ₘₛ or serosa to mucosa *J*ₛₘ. Net flux of Na⁺ is calculated using the equation: *J*ₙₑₜ = *J*ₘₛ - *J*ₛₘ*.* + *J*ₙₑₜ indicates net absorption; whereas -*J*ₙₑₜ indicates net secretion.

In the absence of glucose (0 mM), small intestinal tissues show net sodium absorption (1.8 ± 0.3 mEq.h⁻¹.cm⁻²). Na⁺ absorption is abolished in the presence of 0.6 mM glucose. However, addition of 6 mM glucose results in a significant increase in *Jnet Na*⁺ (3.2 ± 0.5 (µEq.h⁻¹.cm⁻²), indicating net sodium absorption. Unidirectional Na⁺ fluxes do not show significant difference at 0, 0.6 and 6 mM glucose (Fig 2B).

### EXAMPLE 6 - EFFECT OF GLUCOSE ON UNIDIRECTIONAL AND NET FLUX OF CHLORIDE

Change in *I*_{sc} at 0.6 mM glucose is calculated as 1.1 µEq.h⁻¹.cm⁻² (2.2 ± 0.3 - 1.1 ± 0.1 µEq.h⁻¹.cm⁻²) and change in *I*_{sc} at 6 mM glucose is calculated as 2.2 µEq.h⁻¹.cm⁻² (3.4 ± 0.2 - 1.1 ± 0.1 µEq.h⁻¹.cm⁻²). The increase in *I*_{sc} with increasing glucose concentrations cannot be fully explained based on the *J*ₙₑₜ Na⁺ values (based on values at 0.6 and 6 mM glucose).

Isotopic flux measurements for Cl⁻ are performed using ³⁶Cl to determine whether Cl⁻ flux accounts for a portion of the *I*_{sc} that cannot be attributed to *J*ₙₑₜNa⁺. *J*ₙₑₜCl⁻ calculated in the absence of glucose shows Cl⁻ absorption (2 ± 0.3 µEq.h⁻¹.cm⁻²). The level of sodium absorption (1.8 ± 0.3 µEq.h⁻¹.cm⁻²) is comparable to that of chloride (2.0 ± 0.3 µEq.h⁻¹.cm⁻²) in the absence of glucose, indicating electroneutral Na⁺ and Cl⁻ absorption.

Addition of 0.6 mM or 6 mM glucose to the mucosa side results in net Cl⁻ secretion (Fig 2A). *J*ₙₑₜCl⁻ at 0.6 mM glucose (-3.6 ± 0.8 µEq.h⁻¹.cm⁻²) and 6 mM glucose (-4.0 ± 1.4 µEq.h⁻¹.cm⁻²) are not significantly different.

The results show that there is a significant increase in *J*ₛₘCl⁻ in the presence of glucose (at 0.6 and 6 mM glucose) (*J*ₛₘCl⁻ 16.9 ± 0.7 µEq.h⁻¹.cm⁻² and 17 ± 0.7 µEq.h⁻¹.cm⁻², respectively), when compared to *J*ₛₘCl⁻ in the absence of glucose (11.9 ± 0.4 µEq.h⁻¹.cm⁻²) (Fig 2A). The results indicate that significant Cl⁻ secretion occurs at a glucose concentration as low as 0.6 mM. Increasing glucose concentration does not result in increased Cl⁻ secretion.

### EXAMPLE 7 - HCO₃⁻ SECRETION IN ILEUM IN THE ABSENCE OF LUMEN GLUCOSE

Transepithelial electrical measurements and flux studies show that addition of glucose to ileal tissues induces significant Cl⁻-secretion. While *J*ₙₑₜCl⁻ at 0.6 and 6 mM glucose shows significant anion secretion, this does not account for all of the changes in *I*_{sc}, especially in view of the significant differences between *I*_{sc} values at 6 mM glucose 6 µEq.h⁻¹.cm⁻² (7.5 ± 0.4 - 1.5 ± 0.1 µEq.h⁻¹.cm⁻²) and 0.6 mM.

pH stat studies are performed to determine whether HCO₃⁻ secretion contributes to the unaccounted portion of the *I*_{sc}. At least two modes of HCO₃⁻ secretion in the mouse small intestine have been identified by the present inventors: 1) Cl⁻-dependent, electroneutral Cl⁻-HCO₃⁻ exchange, and 2) Cl⁻-independent, electrogenic HCO₃⁻ secretion.

The results show that endogenous HCO₃⁻ secretion does not contribute to net HCO₃⁻ secretion. Specifically, HCO₃⁻-free, poorly buffered solution is added to both sides of the tissues mounted in an Ussing chamber and both sides of the tissues are bubbled with 100% O₂. Minimal HCO₃⁻ secretion (0.1 ± 0.01 mEq.h⁻¹.cm⁻², n=12) is recorded under such conditions. Subsequent addition of HCO₃⁻-containing buffered solution to the basolateral side and bubbling with 95% O₂ and 5% CO₂ on that side results in significant HCO₃⁻ secretion 3.8 ± 0.2 mEq.h⁻¹.cm⁻² (n =9).

To determine whether lumen Cl⁻-independent HCO₃⁻ secretion plays a role in HCO₃⁻ secretion (in the absence of lumen glucose), pH stat experiments are performed in the absence of lumen Cl⁻. In the absence of lumen Cl⁻, minimal HCO₃⁻ secretion is recorded (0.4 ± 0.1 µEq.h⁻¹.cm⁻²) (Fig 5A). The results indicate that the basal HCO₃⁻ secretion in the absence of lumen glucose is primarily due to Cl⁻-dependent, electroneutral Cl⁻-HCO₃⁻ exchange.

### EXAMPLE 8 - EFFECT OF LUMEN GLUCOSE ON HCO₃⁻ SECRETION IN ILEUM

pH stat experiments are performed to determine the effect of glucose on lumen Cl⁻-dependent HCO₃⁻ secretion. In the presence of lumen Cl⁻, addition of glucose to the lumen side results in a significant HCO₃⁻ secretion (7.6 ± µEq.h⁻¹.cm⁻²).

The HCO₃⁻ secretion in the presence of glucose could be due to a lumen Cl⁻-dependent, electroneutral Cl-HCO₃⁻ exchange or a lumen Cl⁻-independent anion channel-mediated HCO₃⁻ secretion. To assess the mechanism of glucose-stimulated HCO₃⁻ secretion, glucose is added to the mucosal side. Removal of lumen Cl⁻ does not abolish HCO₃⁻ secretion in tissues incubated with 6 mM glucose (3.2 ± 0.6 µEq.h⁻¹.cm⁻²) (Fig 5A). The results indicate that HCO₃⁻ secretion in the presence of glucose is primarily due to lumen Cl⁻-independent secretion, and is anion channel-mediated.

In another experiment, 100 µM 5-nitro-2-(3-phenylpropylamino)-benzoic acid (NPPB), a non-specific anion channel blocker, is added to the lumen side. NPPB inhibits lumen Cl⁻-independent HCO₃⁻ secretion detected in the presence of 6 mM glucose (Fig 5B). The results indicate that glucose-stimulated HCO₃⁻ secretion is mediated via an anion channel.

To investigate whether glucose-induced HCO₃⁻ secretion occurs via a CFTR channel, 100 µM glibenclamide is added to the lumen side. Glibenclamide inhibits lumen Cl⁻-independent HCO₃⁻ secretion-stimulated by glucose, indicating that CFTR channels mediate glucose-stimulated HCO₃⁻ secretion.

### EXAMPLE 9 - EFFECT OF GLUCOSE METABOLISM ON ANION CHANNEL-MEDIATED HCO₃⁻ SECRETION

To assess whether glucose metabolism in the small intestine tissue attributes to the glucose-stimulated HCO₃⁻ secretion, small intestine tissues are incubated with 3-OMG, a poorly metabolized form of glucose, in the absence of lumen and bath HCO₃⁻. HCO₃⁻ secretion (0.1 ± 0.03 µEq.h⁻¹.cm⁻²) is observed in the presence of 3-OMG (6 mM) and absence of lumen and bath HCO₃⁻.

### EXAMPLE 10 - EFFECT OF GLUCOSE ON INTRACELLULAR cAMP LEVEL IN ILEUM

In the absence of glucose, cell lysates from the villus cells show a higher intracellular cAMP level, when compared to that of crypt cells. Incubation with forskolin results in a significant increase in [cAMP]ᵢ level in villus and crypt cells (Fig 3A). Forskolin-treated cells are used as a positive control.

To study the effect of glucose on intracellular cAMP levels, the villus and crypt cells are incubated with 6 mM glucose. Incubation of villus cell lysates with glucose results in a significant increase in intracellular cAMP level, when compared to that of crypt cells (Fig 3B). The results indicate that the glucose-mediated increase in intracellular cAMP level plays a role in mediating glucose-stimulated anion secretion. Increased [cAMP]ᵢ is observed in villus cells but not in crypt cells; this indicates that glucose transport machinery is only needed in fully mature and differentiated villus epithelial cells.

To determine whether glucose metabolism has an effect on intracellular cAMP level, mucosal scraping from the ileum is pre-incubated with 3-OMG for 45 minutes and then the cell lysates are used for measuring intracellular cAMP level.

Similar to glucose, incubation of villus cells with 3-OMG at concentrations of 0.6 and 6 mM results in significant increase in intracellular cAMP level (Fig 3C). Incubation of villus cells with 3-OMG at 6 mM results in a significantly higher intracellular cAMP level, when compared to that of 6 mM glucose (*P* < 0.01) (Fig 3C). The results show that the observed increase in intracellular cAMP level is not caused by glucose metabolism in small intestine tissues.

### EXAMPLE 11 - EFFECT OF GLUCOSE ON INTRACELLULAR CA²⁺ IN Caco2 CELL LINES

PKA inhibitor (H-89) inhibits both cAMP-stimulated anion secretion and SGLT1-mediated glucose transport. Presence of H-89-insensitive *I*_{sc} (Fig 1A & B) indicates that PKA-independent mechanisms also contribute to the glucose-induced secretion. As cAMP, intracellular Ca²⁺ is one of the chief intracellular second messengers involved in anion secretion.

To determine the role of intracellular Ca²⁺ in glucose-stimulated increase in *Isc,* intracellular Ca²⁺ level is measured in the presence of different concentrations of glucose and 3-OMG, respectively, and in the presence of BAPTA-AM (1,2-**b**is(o-**a**mino**p**henoxy)ethane-N,N,N',N'-**t**etra**a**cetic acid) - an intracellular calcium-specific chelator. The Ca²⁺ responses to glucose and 3-OMG in cultured Caco2 cells loaded with the Ca²⁺ indicator fluo 8 are monitored by laser scanning confocal microscopy. Addition of 0.6 mM glucose to the bath medium initiates intracellular Ca²⁺ oscillation (Fig 4 B). The amplitude of the oscillations decreases with time. The mean peak amplitude of calcium fluorescence (F/Fo) with 0.6 mM glucose is calculated to be 1.32 ± 0.1 (n=10).

Glucose-induced Ca²⁺ oscillation is not related to the intracellular metabolism of glucose, as 0.6 mM 3-OMG glucose induces similar Ca²⁺ oscillation (1.2 ± 0.1 (n=10) (Fig 4A). Glucose-stimulated Ca²⁺ oscillation is abolished by pre-incubating the cells with intracellular Ca²⁺ chelator BAPTA-AM for 45 minutes (1 .01 ± 0.1) (n=10) (Fig 4A).

Glucose is added at a higher concentration (6 mM) to determine whether increased glucose concentration increases the amplitude of the Ca²⁺ oscillation. The Ca²⁺ oscillations are significantly higher with addition of glucose (1.85 ± 0.2 vs 1.32 ± 0.1) or 3-OMG (1.5 ± 0.1 vs 1.2 ± 0.2) at 6 mM to the bathing medium, when compared to that of 0.6 mM glucose or 3-OMG (Fig 4A). Glucose-stimulated increase in Ca²⁺ oscillations is completely abolished by pre-incubating the cells with BAPTA-AM (Fig 4A). This indicates that intracellular Ca²⁺ is involved in glucose-induced anion secretion.

### EXAMPLE 12 - THERAPEUTIC COMPOSITIONS FOR TREATMENT OF DIARRHEA

In certain embodiments, this Example provides formulations for treating diarrhea, such as rotavirus-induced diarrhea. In one embodiment, the formulation does not comprise glucose, glucose analogs, substrates of glucose transporters, or sugar molecules.

| **Formulation 1** | | |
|---|---|---|
| (Serving Size 1 bottle (237 ml) | | |
| | Amount per serving | % Daily Value* |
| L-Valine | 276 mg * | |
| L-Aspartic Acid | 252 mg * | |
| L-Serine | 248 mg * | |
| L-Isoleucine | 248 mg * | |
| L-Threonine | 225 mg * | |
| L-Lysine HCL | 172 mg * | |
| L-Glycine | 141 mg * | |
| L-Tvrosine | 51 mg * | |
| Other Ingredients: Water, Electrolytes | | |

| **Formulation 2** | | |
|---|---|---|
| (Serving Size 1 bottle (237 ml) | | |
| Amount per serving | | |
| | | % Daily Value * |
| Total Fat 0 g | | 0% |
| Sodium 440 mg | | 18% |
| Total Carbohydrate 0 g | | 0% |
| Protein 2g | | |
| Ingredients: Water, Amino Acids (L-Tryptophan, L-Valine, L-Aspartic Acid, L-Serine, L-Isoleucine, L-Threonine, L-Lysine Hydrochloride, L-Glycine, L-Tyrosine), Electrolytes | | |
| Amino Acid | | Amount mg/1 bottle serving (237 ml) |
| L-Lysine HCI | | 175 |
| L-Aspartic Acid | 255 | |
| L-Glycine | 144 | |
| L-Isoleucine | 251 | |
| L-Threonine | 228 | |
| L-Tyrosine | 52 | |
| L-Valine | 281 | |
| L-Tryptophan | 392 | |
| L-Serine | 252 | |

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (*e.g*., all exact exemplary values provided with respect to a particular factor or measurement can be considered to also provide a corresponding approximate measurement, modified by "about," where appropriate).

The use of any and all examples, or exemplary language (*e.g*., "such as") provided herein, is intended merely to better illuminate the invention. No language in the specification should be construed as indicating any element is essential to the practice of the invention unless as much is explicitly stated.

The description herein of any aspect or embodiment of the invention using terms such as "comprising", "having", "including" or "containing" with reference to an element or elements is intended to provide support for a similar aspect or embodiment of the invention that "consists of', "consists essentially of', or "substantially comprises" that particular element or elements, unless otherwise stated or clearly contradicted by context (*e.g.,* a composition described herein as comprising a particular element should be understood as also describing a composition consisting of that element, unless otherwise stated or clearly contradicted by context).

It should be understood that the examples and embodiments described herein are for illustrative purposes only.

### REFERENCES

Zhang H, Ameen N, Melvin JE, & Vidyasagar S (2007) Acute inflammation alters bicarbonate transport in mouse ileum. J Physiol 581(3):1221-1233.
Hediger MA & Rhoads DB (1994) Molecular physiology of sodium-glucose cotransporters. (Translated from eng) Physiol. Rev. 74(4):993-1026 (in eng).
Wright EM, Loo DD, Hirayama BA, & Turk E (2004) Surprising versatility of Na+-glucose cotransporters: SLC5. (Translated from eng) Physiology (Bethesda) 19:370-376 (in eng).
Chen XZ, Coady MJ, Jackson F, Berteloot A, & Lapointe JY (1995) Thermodynamic determination of the Na+: glucose coupling ratio for the human SGLT1 cotransporter. (Translated from eng) Biophys. J. 69(6):2405-2414 (in eng).
Torres-Pinedo R, Rivera CL, & Fernandez S (1966) Studies on infant diarrhea. II. Absorption of glucose and net fluxes of water and sodium chloride in a segment of the jejunum. (Translated from eng) J. Clin. Invest. 45(12):1916-1922 (in eng).
Davidson GP, Gall DG, Petric M, Butler DG, & Hamilton JR (1977) Human rotavirus enteritis induced in conventional piglets. Intestinal structure and transport. (Translated from eng) J. Clin. Invest. 60(6):1402-1409 (in eng).
Nalin DR, et al. (1979) Oral rehydration and maintenance of children with rotavirus and bacterial diarrhoeas. (Translated from eng) Bull. World Health Organ. 57(3):453-459 (in eng).
Lorrot M & Vasseur M (2007) How do the rotavirus NSP4 and bacterial enterotoxins lead differently to diarrhea? (Translated from eng) Virol J 4:31 (in eng).
Chapman MJ, et al. (2009) Glucose absorption and gastric emptying in critical illness. (Translated from eng) Crit Care 13(4):R140 (in eng).
Telch J, et al. (1981) Intestinal glucose transport in acute viral enteritis in piglets. (Translated from eng) Clin Sci (Lond) 61(1):29-34 (in eng).
Lo B & Silverman M (1998) Cysteine scanning mutagenesis of the segment between putative transmembrane helices IV and V of the high affinity Na+/Glucose cotransporter SGLT1. Evidence that this region participates in the Na+ and voltage dependence of the transporter. (Translated from eng) J. Biol. Chem. 273(45):29341-29351 (in eng).
Wright EM, Hirsch JR, Loo DD, & Zampighi GA (1997) Regulation of Na+/glucose cotransporters. (Translated from eng) J. Exp. Biol. 200(Pt 2):287-293 (in eng).
Dyer J, Vayro S, King TP, & Shirazi-Beechey SP (2003) Glucose sensing in the intestinal epithelium. (Translated from eng) Eur. J. Biochem. 270(16):3377-3388 (in eng).
Gribble FM (2008) RD Lawrence Lecture 2008: Targeting GLP-1 release as a potential strategy for the therapy of Type 2 diabetes. (Translated from eng) Diabet. Med. 25(8):889-894 (in eng).
Gustavsson N, et al. (Synaptotagmin-7 as a positive regulator of glucose-induced glucagon-like peptide-1 secretion in mice. (Translated from eng) Diabetologia 54(7):1824-1830 (in eng).
Eissele R, et al. (1992) Glucagon-like peptide-1 cells in the gastrointestinal tract and pancreas of rat, pig and man. (Translated from eng) Eur. J. Clin. Invest. 22(4):283-291 (in eng).
Elliott RM, et al. (1993) Glucagon-like peptide-1 (7-36)amide and glucose-dependent insulinotropic polypeptide secretion in response to nutrient ingestion in man: acute post-prandial and 24-h secretion patterns. (Translated from eng) J. Endocrinol. 138(1):159-166 (in eng).
Herrmann C, et al. (1995) Glucagon-like peptide-1 and glucose-dependent insulin-releasing polypeptide plasma levels in response to nutrients. (Translated from eng) Digestion 56(2):117-126 (in eng).
Knickelbein RG, Aronson PS, & Dobbins JW (1988) Membrane distribution of sodium-hydrogen and chloride-bicarbonate exchangers in crypt and villus cell membranes from rabbit ileum. J. Clin. Invest. 82(6):2158-2163.
Vidyasagar S, Rajendran VM, & Binder HJ (2004) Three distinct mechanisms of HCO3-secretion in rat distal colon. Am J Physiol Cell Physiol 287(3):C612-621.
Coon S, Kekuda R, Saha P, & Sundaram U (Reciprocal regulation of the primary sodium absorptive pathways in rat intestinal epithelial cells. (Translated from eng) Am J Physiol Cell Physiol 300(3):C496-505 (in eng).
Hu Z, et al. (2006) MAPKAPK-2 is a critical signaling intermediate in NHE3 activation following Na+-glucose cotransport. (Translated from eng) J. Biol. Chem. 281(34):24247-24253 (in eng).
Turner JR & Black ED (2001) NHE3-dependent cytoplasmic alkalinization is triggered by Na(+)-glucose cotransport in intestinal epithelia. (Translated from eng) Am J Physiol Cell Physiol 281(5):C1533-1541 (in eng).
Donowitz M & Li X (2007) Regulatory binding partners and complexes of NHE3. (Translated from eng) Physiol. Rev. 87(3):825-872 (in eng).
Zachos NC, Kovbasnjuk O, & Donowitz M (2009) Regulation of intestinal electroneutral sodium absorption and the brush border Na+/H+ exchanger by intracellular calcium. (Translated from eng) Ann. N. Y. Acad. Sci. 1165:240-248 (in eng).
Zachos NC, et al. (2009) NHERF3 (PDZK1) contributes to basal and calcium inhibition of NHE3 activity in Caco-2BBe cells. (Translated from eng) J. Biol. Chem. 284(35):23708-23718 (in eng).
Donowitz M, et al. (2009) NHE3 regulatory complexes. (Translated from eng) J. Exp. Biol. 212(Pt 11):1638-1646 (in eng).
Zhu X, et al. (Elevated Calcium Acutely Regulates Dynamic Interactions of NHERF2 and NHE3 Proteins in Opossum Kidney (OK) Cell Microvilli. (Translated from eng) J. Biol. Chem. 286(40):34486-34496 (in eng).
Caputo A, et al. (2008) TMEM16A, a membrane protein associated with calcium-dependent chloride channel activity. (Translated from eng) Science 322(5901):590-594 (in eng).
Namkung W, Phuan PW, & Verkman AS (TMEM16A inhibitors reveal TMEM16A as a minor component of calcium-activated chloride channel conductance in airway and intestinal epithelial cells. (Translated from eng) J. Biol. Chem. 286(3):2365-2374 (in eng).
Engle MJ, Goetz GS, & Alpers DH (1998) Caco-2 cells express a combination of colonocyte and enterocyte phenotypes. (Translated from eng) J. Cell. Physiol. 174(3):362-369 (in eng).
Ball JM, Tian P, Zeng CQ, Morris AP, & Estes MK (1996) Age-dependent diarrhea induced by a rotaviral nonstructural glycoprotein. (Translated from eng) Science 272(5258):101-104 (in eng).
Rhoads JM, et al. (1996) Can a super oral rehydration solution stimulate intestinal repair in acute viral enteritis? (Translated from eng) J. Diarrhoeal Dis. Res. 14(3):175-181 (in eng).

## Claims

1. A sterile therapeutic composition for use in a method of treating diarrhea in a subject, wherein the composition is formulated for enteral administration and has a total osmolarity from 100 mosm to 250 mosm,
wherein the composition comprises:
one or more free amino acids selected from lysine, glycine, threonine, valine, tyrosine, aspartic acid, isoleucine, tryptophan, and serine; electrolytes, and water;
and wherein a substrate of a glucose transporter and/or a compound that can be hydrolyzed into a substrate of a glucose transporter, if present in said composition, is present at a concentration of less than 0.05 mM; and,
wherein said substrate of a glucose transporter is glucose, α-methyl-D-glucopyranoside (AMG), 3-O-methylglucose (3-OMG), deoxy-D-glucose, or α-methyl-D-glucose,
and wherein the subject is a human five years old or younger.

2. The composition for use according to claim 1, wherein the diarrhea is rotavirus-induced diarrhea.

3. The composition for use according to claim 1, wherein the composition does not contain any carbohydrate.

4. The composition for use according to claim 1, having a pH of 2.9 to 7.3.

5. The composition for use according to claim 1, wherein the composition is administered orally.

6. The composition for use according to claim 1, wherein the composition does not contain glucose, α-methyl-D-glucopyranoside (AMG), 3-O-methylglucose (3-OMG), deoxy-D-glucose, or α-methyl-D-glucose.

7. The composition for use according to claim 1, wherein the composition comprises one or more electrolytes selected from Na⁺, K⁺, HCO₃⁻, CO₃²⁻, and Cl⁻.

8. The composition for use according to claim 1, wherein the composition consists essentially of
one or more free amino acids selected from aspartic acid, glycine, isoleucine, lysine, serine, threonine, valine, tryptophan, and tyrosine, preferably lysine, aspartic acid, glycine, isoleucine, threonine, tyrosine, valine, and serine;
one or more electrolytes selected from Na⁺, K⁺, HCO₃⁻, CO₃²⁻, and Cl⁻;
water; and,
optionally, one or more carriers, buffering agents, preservatives, and/or flavoring agents.

9. A package containing the composition of claim 1, or a powder which, when combined with a specified amount of water, makes a composition of claim 1 for use according to claim 1.

10. The composition for use according to claim 1, wherein the composition comprises one or more free amino acids selected from lysine, aspartic acid, glycine, isoleucine, threonine, tyrosine, valine, and serine.

11. The composition for use according to claim 8, wherein the composition consists essentially of
aspartic acid, glycine, isoleucine, lysine, serine, threonine, valine, tryptophan, and tyrosine;
one or more electrolytes selected from Na⁺, K⁺, HCO₃⁻, CO₃²⁻, and Cl⁻;
water; and,
optionally, one or more carriers, buffering agents, preservatives, and/or flavoring agents.

12. The composition for use according to claim 8, wherein the composition consists essentially of
lysine, aspartic acid, glycine, isoleucine, threonine, tyrosine, valine, and serine;
one or more electrolytes selected from Na⁺, K⁺, HCO₃⁻, CO₃²⁻, and Cl⁻;
water; and,
optionally, one or more carriers, buffering agents, preservatives, and/or flavoring agents.

13. A sterile therapeutic composition for use in a method of treating diarrhea in a subject, wherein the composition is formulated for enteral administration,
wherein the composition comprises:
one or more free amino acids selected from lysine, glycine, threonine, valine, tyrosine, aspartic acid, isoleucine, tryptophan, and serine; and electrolytes;
and wherein a substrate of a glucose transporter and/or a compound that can be hydrolyzed into a substrate of a glucose transporter, if present in said composition, is present at a concentration of less than 0.05 mM; and,
wherein said substrate of a glucose transporter is glucose, α-methyl-D-glucopyranoside (AMG), 3-O-methylglucose (3-OMG), deoxy-D-glucose, or α-methyl-D-glucose,
and wherein the subject is a human five years old or younger;
wherein, when the composition is reconstituted in water for use in said method, the composition has a total osmolarity from 100 mosm to 250 mosm.

## Patentansprüche

1. Sterile therapeutische Zusammensetzung zur Verwendung in einem Verfahren zur Behandlung von Durchfall in einem Subjekt, wobei die Zusammensetzung für die enterale Verabreichung formuliert ist und eine Gesamtosmolarität von 100 mosm bis 250 mosm aufweist,
wobei die Zusammensetzung Folgendes umfasst:
eine oder mehrere freie Aminosäuren ausgewählt aus Lysin, Glycin, Threonin, Valin, Tyrosin, Asparaginsäure, Isoleucin, Tryptophan und Serin; Elektrolyte und Wasser;
und wobei ein Substrat eines Glucosetransporters und/oder einer Verbindung, die zu einem Substrat eines Glucosetransporters hydrolysiert werden kann, wenn in der Zusammensetzung vorhanden, in einer Konzentration von weniger als 0,05 mM vorliegt und
wobei es sich bei dem Substrat eines Glucosetransporters um Glucose, α-Methyl-D-glucopyranosid (AMG), 3-O-Methylglucose (3-OMG), Deoxy-D-glucose oder α-Methyl-D-glucose handelt,
und wobei es sich bei dem Subjekt um einen Menschen mit einem Alter von 5 Jahren oder jünger handelt.

2. Zusammensetzung zur Verwendung nach Anspruch 1, wobei es sich bei dem Durchfall um rotavirusinduzierten Durchfall handelt.

3. Zusammensetzung zur Verwendung nach Anspruch 1, wobei die Zusammensetzung keine Kohlenhydrate enthält.

4. Zusammensetzung zur Verwendung nach Anspruch 1 mit einem pH-Wert von 2,9 bis 7,3.

5. Zusammensetzung zur Verwendung nach Anspruch 1, wobei die Zusammensetzung oral verabreicht wird.

6. Zusammensetzung zur Verwendung nach Anspruch 1, wobei die Zusammensetzung weder Glucose, α-Methyl-D-glucopyranosid (AMG), 3-O-Methylglucose (3-OMG), Deoxy-D-glucose noch α-Methyl-D-glucose enthält.

7. Zusammensetzung zur Verwendung nach Anspruch 1, wobei die Zusammensetzung einen oder mehrere Elektrolyte ausgewählt aus Na⁺, K⁺, HCO₃⁻, CO₃²⁻ und Cl⁻ umfasst.

8. Zusammensetzung zur Verwendung nach Anspruch 1, wobei die Zusammensetzung im Wesentlichen aus Folgendem besteht:
einer oder mehreren freien Aminosäuren ausgewählt aus Asparaginsäure, Glycin, Isoleucin, Lysin, Serin, Threonin, Valin, Tryptophan und Tyrosin, vorzugsweise Lysin, Asparaginsäure, Glycin, Isoleucin, Threonin, Tyrosin, Valin und Serin,
einem oder mehreren Elektrolyten ausgewählt aus Na⁺, K⁺, HCO₃⁻, CO₃²⁻ und Cl⁻,
Wasser und
gegebenenfalls einem oder mehreren Trägern, Puffern, Konservierungsstoffen und/oder Geschmacksstoffen.

9. Packung, enthaltend die Zusammensetzung nach Anspruch 1 oder ein Pulver, das bei Kombination mit einer spezifizierten Menge an Wasser eine Zusammensetzung nach Anspruch 1 liefert, zur Verwendung nach Anspruch 1.

10. Zusammensetzung zur Verwendung nach Anspruch 1, wobei die Zusammensetzung eine oder mehrere freie Aminosäuren ausgewählt aus Lysin, Asparaginsäure, Glycin, Isoleucin, Threonin, Tyrosin, Valin und Serin umfasst.

11. Zusammensetzung zur Verwendung nach Anspruch 8, wobei die Zusammensetzung im Wesentlichen aus Folgendem besteht:
Asparaginsäure, Glycin, Isoleucin, Lysin, Serin, Threonin, Valin, Tryptophan und Tyrosin,
einem oder mehreren Elektrolyten ausgewählt aus Na⁺, K⁺, HCO₃⁻, CO₃²⁻ und Cl⁻,
Wasser und
gegebenenfalls einem oder mehreren Trägern, Puffern, Konservierungsstoffen und/oder Geschmacksstoffen.

12. Zusammensetzung zur Verwendung nach Anspruch 8, wobei die Zusammensetzung im Wesentlichen aus Folgendem besteht:
Lysin, Asparaginsäure, Glycin, Isoleucin, Threonin, Tyrosin, Valin und Serin,
einem oder mehreren Elektrolyten ausgewählt aus Na⁺, K⁺, HCO₃⁻, CO₃²⁻ und Cl⁻,
Wasser und
gegebenenfalls einem oder mehreren Trägern, Puffern, Konservierungsstoffen und/oder Geschmacksstoffen.

13. Sterile therapeutische Zusammensetzung zur Verwendung in einem Verfahren zur Behandlung von Durchfall in einem Subjekt, wobei die Zusammensetzung für die enterale Verabreichung formuliert ist,
wobei die Zusammensetzung Folgendes umfasst:
eine oder mehrere freie Aminosäuren ausgewählt aus Lysin, Glycin, Threonin, Valin, Tyrosin, Asparaginsäure, Isoleucin, Tryptophan und Serin; und Elektrolyte;
und wobei ein Substrat eines Glucosetransporters und/oder einer Verbindung, die zu einem Substrat eines Glucosetransporters hydrolysiert werden kann, wenn in der Zusammensetzung vorhanden, in einer Konzentration von weniger als 0,05 mM vorliegt und
wobei es sich bei dem Substrat eines Glucosetransporters um Glucose, α-Methyl-D-glucopyranosid (AMG), 3-O-Methylglucose (3-OMG), Deoxy-D-glucose oder α-Methyl-D-glucose handelt,
und wobei es sich bei dem Subjekt um einen Menschen mit einem Alter von 5 Jahren oder jünger handelt,
wobei die Zusammensetzung bei Rekonstitution in Wasser zur Verwendung in dem Verfahren eine Gesamtosmolarität von 100 mosm bis 250 mosm aufweist.

## Revendications

1. Composition thérapeutique stérile pour utilisation dans un procédé de traitement de la diarrhée chez un sujet, où la composition est formulée pour administration entérale et a une osmolarité totale de 100 mosm à 250 mosm,
où la composition comprend :
un ou plusieurs acides aminés libres choisis parmi lysine, glycine, thréonine, valine, tyrosine, acide aspartique, isoleucine, tryptophane et sérine ; des électrolytes et de l'eau ;
et dans laquelle un substrat d'un transporteur de glucose et/ou un composé qui peut être hydrolysé en un substrat d'un transporteur de glucose, s'il est présent dans ladite composition, est présent à une concentration inférieure à 0,05 mM ; et,
dans laquelle ledit substrat d'un transporteur de glucose est le glucose, le α-méthyl-D-glucopyranoside (AMG), le 3-O-méthylglucose (3-OMG), le désoxy-D-glucose ou le α-méthyl-D-glucose,
et où le sujet est un humain âgé de cinq ans ou plus jeune.

2. Composition pour utilisation selon la revendication 1, où la diarrhée est une diarrhée induite par un rotavirus.

3. Composition pour utilisation selon la revendication 1, où la composition ne contient aucun glucide.

4. Composition pour utilisation selon la revendication 1, ayant un pH de 2,9 à 7,3.

5. Composition pour utilisation selon la revendication 1, où la composition est administrée par voie orale.

6. Composition pour utilisation selon la revendication 1, où la composition ne contient pas de glucose, α-méthyl-D-glucopyranoside (AMG), 3-O-méthylglucose (3-OMG), désoxy-D-glucose ou α-méthyl-D-glucose.

7. Composition pour utilisation selon la revendication 1, où la composition comprend un ou plusieurs électrolytes choisis parmi Na⁺, K⁺, HCO₃⁻, CO₃²⁻ et Cl⁻.

8. Composition pour utilisation selon la revendication 1, où la composition est essentiellement constituée de
un ou plusieurs acides aminés libres choisis parmi acide aspartique, glycine, isoleucine, lysine, sérine, thréonine, valine, tryptophane et tyrosine, de préférence lysine, acide aspartique, glycine, isoleucine, thréonine, tyrosine, valine et sérine ;
un ou plusieurs électrolytes choisis parmi Na⁺, K⁺, HCO₃⁻, CO₃²⁻ et Cl⁻ ;
de l'eau ; et,
facultativement, un ou plusieurs véhicules, agents tampons, conservateurs et/ou agents aromatisants.

9. Emballage contenant la composition selon la revendication 1, ou une poudre qui, lorsqu'elle est combinée avec une quantité d'eau spécifiée, produit une composition selon la revendication 1 pour utilisation selon la revendication 1.

10. Composition pour utilisation selon la revendication 1, où la composition comprend un ou plusieurs acides aminés libres choisi parmi lysine, acide aspartique, glycine, isoleucine, thréonine, tyrosine, valine et sérine.

11. Composition pour utilisation selon la revendication 8, où la composition est essentiellement constituée de
acide aspartique, glycine, isoleucine, lysine, sérine, thréonine, valine, tryptophane et tyrosine ;
un ou plusieurs électrolytes choisis parmi Na⁺, K⁺, HCO₃⁻, CO₃²⁻ et Cl⁻ ;
de l'eau ; et,
facultativement, un ou plusieurs véhicules, agents tampons, conservateurs et/ou agents aromatisants.

12. Composition pour utilisation selon la revendication 8, la composition étant essentiellement constituée de
lysine, acide aspartique, glycine, isoleucine, thréonine, tyrosine, valine et sérine ;
un ou plusieurs électrolytes choisis parmi Na⁺, K⁺, HCO₃⁻, CO₃²⁻ et Cl⁻ ;
de l'eau ; et,
facultativement, un ou plusieurs véhicules, agents tampons, conservateurs et/ou agents aromatisants.

13. Composition thérapeutique stérile pour utilisation dans un procédé de traitement de la diarrhée chez un sujet, où la composition est formulée pour administration entérale,
où la composition comprend :
un ou plusieurs acides aminés libres choisis parmi les lysine, glycine, thréonine, valine, tyrosine, acide aspartique, isoleucine, tryptophane et sérine ; et des électrolytes ;
et dans laquelle un substrat d'un transporteur de glucose et/ou un composé qui peut être hydrolysé en un substrat d'un transporteur de glucose, s'il est présent dans ladite composition, est présent à une concentration inférieure à 0,05 mM ; et,
dans laquelle ledit substrat d'un transporteur de glucose est le glucose, le α-méthyl-D-glucopyranoside (AMG), le 3-O-méthylglucose (3-OMG), le désoxy-D-glucose ou le α-méthyl-D-glucose,
et où le sujet est un humain âgé de cinq ans ou plus jeune ;
où, lorsque la composition est reconstituée dans de l'eau pour utilisation dans ledit procédé, la composition a une osmolarité totale de 100 mosm à 250 mosm.
